## Europäisches Patentamt

(19) **European Patent Office**

**Office européen des brevets**

(11) Publication number: **0 275 194**
**A2**

(12) # EUROPEAN PATENT APPLICATION

(21) Application number: **88300256.0**

(22) Date of filing: **13.01.88**

㊿ . Int. Cl.⁴: **A 61 K 37/02**
**A 61 K 37/43**

(30) Priority: **14.01.87 US 3160**

(43) Date of publication of application:
**20.07.88 Bulletin 88/29**

(84) Designated Contracting States:
**AT BE CH DE ES FR GB GR IT LI LU NL SE**

(71) Applicant: **IVAX LABORATORIES INCORPORATED**
**8800 N.W. 36th Street**
**Miami Florida 33166 (US)**

(72) Inventor: **Solomon, Samuel**
**Royal Victoria Hospital Room L205**
**685 Pine Avenue West Montreal H3A 1A1 (CA)**

(74) Representative: **Ritter, Stephen David et al**
**Mathys & Squire 10 Fleet Street**
**London EC4Y 1AY (GB)**

(54) **Adrenocorticotrophic hormone inhibiting peptides.**

(57) A method inhibiting adrenocorticotropic hormone in a patient is discosed which comprises administering to the patient a peptide selected from the group consisting of Gly-Ile-Cys-Ala-Cys-Arg-Arg-Arg-Phe-Cys-Pro-Asn-Ser-Glu-Arg-Phe-Ser-Gly-Tyr-Cys-Arg-Val-Asn-Gly-Ala-Arg-Tyr-Val-Arg-Cys-Cys-Ser-Arg-Arg. Pharmaceutical compositions containing and methods for administration of the adrenocorticotrophic hormone inhibiting peptide are also discussed.

**Description**

## ADRENOCORTICOTROPHIC HORMONE INHIBITING PEPTIDES

Adrenocortiocotrophic hormone is involved in several disease conditions. For example, pituitary and ectopic tumors make too much adrenocorticotrophic hormone. The overabundance of the hormone results, for example, in Cushings disease. Thus, compounds having the ability to inhibit adrenocorticotrophic hormone formation are therapeutically useful.

Also, compounds having the ability to inhibit hormone production are also useful in the monitoring of gland function in the gland which produces the hormone. That is, adrenal gland function can be tested through the use of the adrenocorticotrophic hormone inhibiting protein.

In a first aspect of the invention there is provided a method of inhibiting adrenocorticotrophic hormone in a patient which comprises administration of a peptide of the formula Gly-Ile-Cys-Ala-Cys-Arg-Arg-Arg-Phe-Cys-Pro-Asn-Ser-Glu-Arg-Phe-Ser-Gly-Tyr-Cys-Arg-Val-Asn-Gly-Ala-Arg-Tyr-Val-Arg-Cys-Cys-Ser-Arg-Arg.

In one preferred embodiment, said peptide is administered via intravenous infusion. In a second embodiment, said peptide is administered via a tablet dosage form.

In accordance with a second aspect of the present invention, there is provided an anti-adrenocorticotrophic hormone composition for administration to patients which comprises an isotonic solution containing an effective amount of a peptide capable of inhibiting adrenocorticotrophic hormone; and pharmaceutically inert ingredients, said peptide having formula Gly-Ile-Cys-Ala-Cys-Arg-Arg-Arg-Phe-Cys-Pro-Asn-Ser-Glu-Arg-Phe-Ser-Gly-Tyr-Cys-Arg-Val-Asn-Gly-Ala-Arg-Tyr-Val-Arg-Cys-Cys-Ser-Arg-Arg.

An isotonic solution of the invention may contain in addition to said peptide, water and salt, also conventional ingredients such as glucose.

In accordance with a third aspect of the present invention, there is provided an anti-adrenocortiocotrophic hormone composition for administration to patients which comprises an effective amount of a peptide capable of inhibiting adrenocorticotrophic hormine, pharmaceutically acceptable excipients, and pharmaceutically inert ingredients in tablet form, said peptide having formula Gly-Ile-Cys-Ala-Cys-Arg-Arg-Arg-Phe-Cys-Pro-Asn-Ser-Glu-Arg-Phe-Ser-Gly-Tyr-Cys-Arg-Val-Asn-Gly-Ala-Arg-Tyr-Val-Arg-Cys-Cys-Ser-Arg-Arg. Typical tablet dosage form excipients and carriers are contemplated insofar as they do not interfere with the desired biological activity.

The production of substantially pure peptides of the formula Gly-Ile-Cys-Ala-Cys-Arg-Arg-Arg-Phe-Cys-Pro-Asn-Ser-Glu-Arg-Phe-   Ser-Gly-Tyr-Cys-Arg-Val-Asn-Gly-Ala-Arg-Tyr-Val-Arg-Cys-Cys-Ser-Arg-Arg comprises

    (a) extracting rabbit adult and fetal lung tissue in an acid medium;

    (b) separating protein from said peptides via chromatography; and

    (c) sequentially submitting the extract to a series of column purifications which contain respectively

        (i) trifluoroacetic acid;

        (ii) heptafluorobenzoic acid; and

        (iii) trifluoroacetic acid.

The method of application of the adrenocortiocotrophic hormone inhibitor is not of primary importance, it being understood that any method that will provide the adrenocortiocotrophic hormone inhibiting peptide in a sufficiently large concentration to the patient will be satisfactory. Intravenous delivery of peptides is well known as a method of introducing peptides into the bloodstream without destruction in either the gastrointestinal tract or the liver. As practical matter, there are well recognized problems with the oral delivery of peptides, although an oral form that would be capable of delivery of the adrenocortiocotrophic. hormone inhibiting peptide to the bloodstream in a sufficient quantity is contemplated as an aspect of the invention.

An effective amount of adrenocorticotrophic hormone inhibiting peptide in an isotonic solution to be introduced by intravenous infusion is from about 1.5 to about 1500 micrograms/150 kilograms of body weight of the patient, and preferably about 15 to about 150 micrograms/150 kilograms of body weight of the patient.

An effective amount of adrenocortiocotrophic hormone inhibiting peptide in a tablet to be administered orally four times daily is from about 0.37 to about 370 micrograms/150 kilograms of body weight of the patient, and preferable about 3.7 to 37 micrograms/150 kilograms of body weight of the patient.

### EXAMPLE I

The adrenocorticotrophic hormone inhibiting peptide is produced by extraction procedures designed to optimize recovery of peptides rather than proteins. 1.35 grams of frozen rabbit fetal lung tissue is thawed to 4°C and homogenized in an acidic medium which comprises

1 M HCl

5% formic acid

1% NaCl

1% trifluoroacetic acid

and centrifuged at 300 RPM for 15 minutes. The pellet is reextracted two times and the pooled supernatant was concentrated onto ODS cartridges (Sep-Pak C18 cartridges, Waters Associates, Massachusetts), and the peptides are eluted from each cartridge with 4 ml of 80% acetonitrile containing 0.1% trifluoroacetic acid.

Reverese phase HPLC purification of this extract is accomplished with a Waters C-18 μBondapak column using a one hour gradient from 0% to 12% acetonitrile in 0.1% trifluoroacetic acid at 1.5 ml/min followed by another one hour gradient from 12-24% acetonitrile in 0.1% trifluoroacetic acid. The peak fractions are further purified using a gradient of 0% to 80% acetonitrile in 0.13% heptafluorobenzoic acid at 1.5 ml/min. A further purification is performed using the 0.1% trifluoroacetic acid/acetonitrile solvent system of Bennett et al, Biochemistry, 20, pp. 4530-4538 (1981).

EXAMPLE 2

Using the adrenocorticotrophic hormone inhibiting peptide produced in Example I, a solution is prepared for use in intravenous infusion of the peptide. Sufficient amount of adrenocorticotrophic hormone inhibiting peptide is introduced into an isotonic solution to create a 3 mmolar solution wherein the total daily dosage administered is 25 micrograms of adrenocortiocotrophic hormone inhibiting peptide/150 kilograms of the body weight of the patient.

EXAMPLE 3

Using the adrenocorticotrophic hormone inhibiting peptide produced in Example I, a tablet is prepared for oral administration. Sufficient amount of the adrenocorticotrophic hormone inhibiting peptide is admixed with acceptable carriers and excipients and formed into an oral dosage unit of acceptable size capable of delivering about 5 micrograms of adrenocorticotrophic hormone inhibiting peptide/150 kilograms of body weight of the patient into the blood stream of the patient.

EXAMPLE 4

A method of inhibiting the production of adrenocorticotrophic hormone is practiced through the oral administration of the dosage unit of Example 3 four times daily.

In addition to the dosage forms specifically set forth, other forms which would provide the adrenocorticotrophic hormone inhibiting peptide to the bloodstream of a patient without substantial destruction in the gastrointestinal tract or the liver would also be contemplated as an embodiment of the invention.

Claims

1. A method of inhibiting adrenocorticotrophic hormone in a patient which comprises administration of a peptide of the formula
Gly-Ile-Cys-Ala-Cys-Arg-Arg-Arg-Phe-Cys-Pro-Asn-Ser-Glu-Arg-Phe-Ser-Gly-Tyr-Cys-Arg-Val-Asn-Gly-Ala-Arg-Tyr-Val-Arg-Cys-Cys-Ser-Arg-Arg,
preferably by intravenous infusion or in tablet form.

2. An anti-adrenocorticotrophic hormone composition for administration to a patient which comprises an isotonic solution containing an effective amount of a peptide capable of inhibiting adrenocorticotrophic hormone; and pharmaceutically inert ingredients, said peptide having the formula Gly-Ile-Cys-Ala-Cys-Arg-Arg-Arg-Phe-Cys-Pro-Asn-Ser-Glu-Arg-Phe-Ser-Gly-Tyr-Cys-Arg-Val-Asn-Gly-Ala-Arg-Tyr-Val-Arg-Cys-Cys-Ser-Arg-Arg.

3. An anti-adrenocorticotrophic hormone composition for administration to a patient which comprises an effective amount of a peptide capable of inhibiting adrenocorticotrophic hormone, pharmaceutically acceptable excipients, and pharmaceutically inert ingredients in tablet form, said peptide having formula
Gly-Ile-Cys-Ala-Cys-Arg-Arg-Arg-Phe-Cys-Pro-Asn-Ser-Glu-Arg-Phe-Ser-Gly-Tyr-Cys-Arg-Val-Asn-Gly-Ala-Arg-Tyr-Val-Arg-Cys-Cys-Ser-Arg-Arg.

4. The use of peptide of formula
Gly-Ile-Cys-Ala-Cys-Arg-Arg-Arg-Phe-Cys-Pro-Asn-Ser-Glu-Arg-Phe-Ser-Gly-Tyr-Cys-Arg-Val-Asn-Gly-Ala-Arg-Tyr-Val-Arg-Cys-Cys-Ser-Arg-Arg,
in the manufacturing of a pharmaceutical prepartion for inhibiting adrenocorticotrophic hormone in a patient.